# EUROPÄISCHE PATENTSCHRIFT

(11) **EP 0 681 853 B1**
(45) Veröffentlichungstag und Bekanntmachung des Hinweises auf die Patenterteilung: **26.11.1997**
(21) Anmeldenummer: 95105558.1
(22) Anmeldetag: 12.04.1995
(51) Int. Cl.: A63B 23/18

(54) **Atmungstherapiegerät**
Apparatus for respiration therapy
Appareil thérapeutique respiratoire

(30) Priorität: 11.05.1994 DE 4416575
(43) Veröffentlichungstag der Anmeldung: 15.11.1995
(73) Patentinhaber: Cegla, Ulrich H. Prof. Dr. med., D-56140 Montabaur (DE)
(72) Erfinder: Cegla, Ulrich H. Prof. Dr. med., D-56140 Montabaur (DE)
(74) Vertreter: Engelhardt, Guido, Dipl.-Ing.

(56) Entgegenhaltungen:
- US-A- 1 392 700
- US-A- 3 936 048
- US-A- 4 062 358

## Beschreibung

Die Erfindung bezieht sich auf ein Therapiegerät zur Unterstützung der Atmung und der Expektoration eines Patienten, mittels dem beim Aus- oder Einatmen ein oszillierender Luftwiderstand erzeugbar ist.

Durch die EP-B1-33 79 90 ist ein Gerät dieser Art zur Unterstützung der physikalischen Atemtherapie, und zwar zur Belüftung der Lungenperipherie bei Erkrankungen wie chronischer Bronchitis, Bronchiektasen, Mukoviszidose, Lungenemphysem und dgl. bekannt. Dieses Widerstandsgerät weist einen ersten rohrförmigen Abschnitt, der mit einer Lufteinlaßöffnung versehen ist, in die der Patient ausatmen kann, und einen zweiten mit einem kreisförmigen konischen Auslaßkanal versehenen Rohrabschnitt auf, der nach oben abgeknickt ist. In den Auslaßkanal ist hierbei eine Kugel lose eingelegt, deren Durchmesser größer bemessen ist als der kleinste Durchmesser des Auslaßkanals. Vor dem Ausatmen ist somit der Auslaßkanal durch die Kugel verschlossen, durch die von dem Patient ausgeatmete Luft wird die Kugel jedoch angehoben und setzt dabei aufgrund ihres Eigengewichtes dem Ausatmen einen Widerstand entgegen.

Durch den beim Aus atmen erzeugten Luftdruck wird die Kugel bei diesem Gerät zwar hin- und herbewegt, so daß Vibrationen und ein positiver Ausatemwiderstand erzeugt werden. Als nachteilig hat sich jedoch gezeigt, daß der Auslaßkanal durch die Kugel oftmals nicht verschlossen wird. Der erste Rohrabschnitt muß nämlich bei Verwendung dieses Gerätes von dem Patienten etwa horizontal gehalten werden, und zwar derart, daß der zweite Rohrabschnitt vertikal nach oben gerichtet ist, andernfalls ist eine einwandfreie Funktion nicht gewährleistet, die Handhabung ist demnach erschwert. Und da bei zunehmender Atmung der dabei erzeugte Atemdruck abnimmt, wird mitunter der Auslaßkanal vorzeitig verschlossen, so daß trotz der physikalischen Maßnahme am Ende der Ausatmung die Bronchien ebenfalls verschlossen werden.

Aufgabe der Erfindung ist es daher, ein Therapiegerät der vorgenannten Gattung zu schaffen, das nicht nur leicht zu handhaben und in jeder Lage verwendbar ist, sondern mit dem auch intrathorakale Perkussionen erzeugt werden können. Der konstruktive Aufbau des Gerätes soll einfach sein, so daß auch eine wirtschaftliche Herstellung ermöglicht wird, vor allem aber soll erreicht werden, daß während der Ausatmung ein oszillierend positiver Ausatemdruck gegeben ist und daß unterschiedliche Frequenzen leicht eingestellt und demnach an einen Patienten angepaßt werden können.

Gemäß der Erfindung wird dies bei einem Therapiegerät der vorgenannten Art dadurch erreicht, daß an einem Mundstück ein elastisch verformbares Schlauchstück vorzugsweise auswechselbar befestigt ist, das an seinem freien Ende offen ausgebildet ist, und daß das Mundstück in ein gekrümmt oder abgeknickt gestaltetes Rohrstück eingesetzt ist, derart, daß das Schlauchstück an dessen Innenmantelfläche anliegt.

Zweckmäßig ist es hierbei, das Mundstück in Achsrichtung des Rohrstückes verstellbar und/oder drehbar in diesem anzuordnen. Dies kann in einfacher Weise dadurch bewerkstelligt werden, in dem auf der Außenmantelfläche des Mundstückes mit Abstand zueinander angeordnete Ausnehmungen in Form von Rillen, Kerben oder dgl. eingearbeitet werden und daß an dem das Mundstück aufnehmenden Ende des Rohrstückes ein oder mehrere gleichmäßig über den Umfang verteilt angeordnete, z. B. als Rastnasen ausgebildete Vorsprünge vorgesehen werden, die in die Ausnehmungen eingreifen.

Das an dem Mundstück angebrachte Schlauchstück sollte eine rechteckige Querschnittsfläche aufweisen und in sich elastisch verformbar sein, auch sollte das an dem Mundstück angebrachte Schlauchstück in dem an der Innenmantelfläche des Rohrstückes anliegenden Bereich eine Abwinkelung aufweisen.

Angebracht ist es ferner, in das freie Ende des Rohrstückes ein weiteres Mundstück einzusetzen oder das freie Ende des Rohrstückes mit einer luftdurchlässigen oder teilweise offenen Verschlußkappe zu versehen.

Wird ein Therapiegerät gemäß der Erfindung ausgebildet und zur Unterstützung der Atmung und der Expektoration eines Patienten eingesetzt, so ist es möglich, durch intrathorakale Vibrationen den Bronchialschleim zu verflüssigen, ohne daß bei forcierter Ausatmung durch den im Brustkorb entstehenden Druck die Bronchien bzw. die Luftröhre verschlossen werden. Beim Ausatmen und somit beim Anblasen des in dem gekrümmten Rohrstück eingesetzten sich abknickenden Schlauchstückes entsteht vielmehr ein oszillierend positiver Druck, außerdem vibriert das freie Ende des Schlauchstückes im niederfrequenten im niederfrequenten Bereich, so daß trotz der Vibration und trotz des positiven Ausatemwiderstandes eine genügend hohe Flußgeschwindigkeit im Bronchialbaum herrscht und der gelöste und verflüssigte Schleim auch aus den Bronchien entfernt werden kann.

Von Vorteil ist hierbei des weiteren, daß über die gesamte Ausatmung ein oszillierend positiver Ausatemdruck besteht und daß durch Verändern der Lage des Mundstückes zu dem Rohrstück unterschiedliche Frequenzen leicht eingestellt werden können. Auch kann durch die Krümmung des Rohrstückes sowie die Länge des Schlauchstückes die Frequenz verändert werden. Außerdem kann durch die Wahl der Abmessungen der einzelnen Bauteile des Therapiegerätes sowie durch die Auswahl des jeweiligen Werkstoffes die Vibrations-Amplitude beeinflußt werden, so daß die intrathorakalen Perkussionen in den Bereich der Resonanzfrequenz des Brustkorbes zwischen 12 und 30 Hz, in dem diese besonders wirksam sind, gelegt und fest eingestellt werden können. Durch Verwendung verschiedenartiger Schlauchstücke kann auch der Ausatemdruck und die Vibrationsmasse in kurzer Zeit und ohne Schwierigkeiten variiert werden. Bei einfacher problemloser Handhabung ist das vorschlagsgemäß ausgebildete Therapiegerät somit vielseitig und vorteilhaft einsetzbar.

Weitere Lösungsmerkmale der Erfindung ergeben sich aus den Unteransprüchen.

In der Zeichnung ist ein Ausführungsbeispiel des gemaß der Erfindung ausgebildeten Therapiegerätes zur Unterstützung der Atmung eines Patienten dargestellt, das nachfolgend im einzelnen erläutert ist. Hierbei zeigt:
- Figur 1: das aus einem Mundstück, einem an diesem angebrachten Schlauchstück sowie einem gekrümmten Rohrstück bestehende Therapiegerät, in einem Längsschnitt,
- Figur 2: einen Schnitt nach der Linie II - II der Figur 1, und
- Figur 3: das Therapiegerät nach Figur 1 mit einem zweiten in das Rohrstück eingestzten Mundstück.

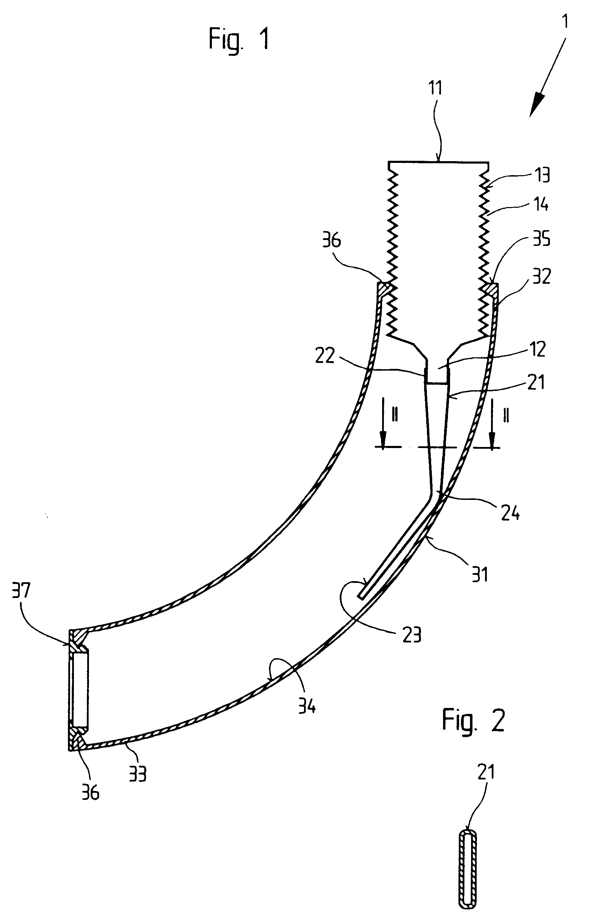

Das in Figur 1 dargestellte und mit 1 bezeichnete Therapiegerät dient zur Unterstützung der Atmung und der Expektoration eines Patienten durch Perkussionen sowie zur Belüftung der Lungenperipherie bei Erkrankungen wie chronischer Bronchitis, Bronchiektasen, Mukoviszidose, Lungenemphysem oder dgl. und besteht im wesentlichen aus einem Mundstück 11, an dem mittels eines angeformten Ansatzes 12 ein Schlauchstück 21 lösbar befestigt ist, sowie einem gekrümmten Rohrstück 31, in dessen eines Ende 32 das Mundstück 11 eingesetzt ist. Das andere Ende 33 des Rohrstückes 31 ist mit einer luftdurchlässigen Verschlußkappe 37 abgedeckt.

Um den Bronchialschleim durch intrathorakale Vibrationen zu lösen und diese Perkussionen leicht auf die Resonanzfrequenz des Brustkorbes, die zwischen 12 und 30 Hz liegt, einstellen zu können, ist das Mundstück 11 in dem Rohrstück 31 verstellbar eingesetzt. Dazu sind in das Mundstück 11 mit Abstand zueinander angeordnete Ausnehmungen 13 in Form von Rillen 14 eingearbeitet und an der Innenmantelfläche 34 des Rohrstückes 31 sind im Bereich der beiden Enden 32 und 33 Vorsprünge 35 nach Art von Rastnasen 36 angebracht, die in die Rillen 14 des Mundstückes 11 eingreifen. Des weiteren ist das mit dem einen Ende 22 an dem Ansatz 12 auswechselbar befestigte Schlauchstück 21, dessen anderes Ende 23 offen ausgebildet ist, mit einer Abwinkelung 24 versehen, die sich durch Anlage an der Innenwandung 34 des Rohrstückes 31 ausbildet.

Wird das Therapiegerät 1 von einem Patienten benutzt und somit beim Ausatmen dem einen rechteckigen Querschnitt aufweisenden und sich elastisch verformbaren Schlauchstück 21 Atemluft zugeführt, so wird durch das Therapiegerät 1 ein oszillierend positiver Druck erzeugt, außerdem beginnt das freie Ende 23 des Schlauchstückes 21 niederfrequent im Bereich der Resonanzfrequenz des Brustkorbes zu vibrieren. Die Frequenz und der Durchblasdruck sind durch Verstellen des Mundstückes 11 beeinflußbar, so daß diese an den Patienten angepaßt werden kann und Schleim in den Bronchien gelöst wird, so daß die Lungenventilation verbessert und das Abhusten bei obstruktiven Lungenerkrankungen erleichtert wird.

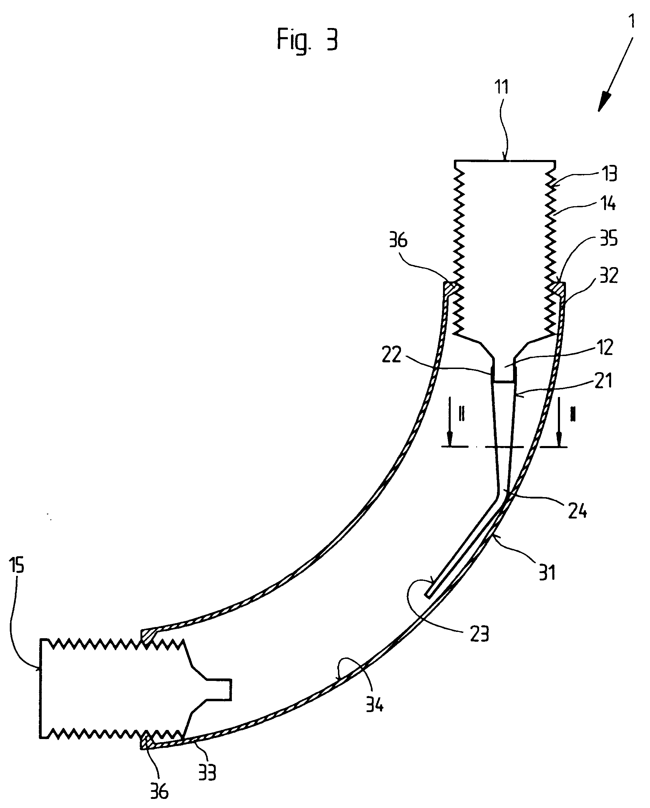

Um das Therapiegerät 1 auch bei der Einatmung benutzen zu können, ist, wie dies in Figur 3 dargestellt ist, an dem dem Mundstück 21 gegenüberliegenden Ende 33 des Rohrstückes 31 ein weiteres Mundstück 15 in dieses eingesetzt. Die einzuatmende Luft wird in diesem Fall über das sich öffnende Schlauchstück 21 angesaugt. Bei diesem Einatmungsvorgang entstehen Vibration- und Druckänderungen, die denen entsprechen, die beim Ausatmen durch das Therapiegerät 1 entstehen.

Es ist ebenfalls vorstellbar, das Therapiegerät 1 sowohl für die Einatmung als auch für die Ausatmung zu verwenden. Dazu wird das Therapiegerät 1 nach Figur 3 eingesetzt.

Über das Mundstück 15 wird hierbei die Luft durch das Schlauchstück 21 angesaugt. Dabei liegt das Schlauchstück 21 durch entsprechendes Ein- und/oder Verdrehen des Mundstückes 11 an dem gekrümmten Rohrstück 31 an und vibriert mit einer bestimmten Frequenz.

Vor dem Ausatmen muß der Benutzer das Therapiegerät 1' umdrehen und die in der Lunge gespeicherte Luft durch das Mundstück 11 durch das Schlauchstück 21 hindurchdrücken.

Dieser Saug-Druck-Effekt beim Aus- und Einatmen durch das Therapiegerät 1' verstärkt das Ausdauermuskeltraining der Einatmungsmuskulatur. Des weiteren führen die Vibrationen beim Ein- und Ausatmen zu einer Verflüssigung des Bronchialschleims, wobei gleichzeitig durch die entstandenen Vibrationen mögliche Schleimpfropfen in den- Bereich der Lunge zurückgezogen werden, in dem noch eine oberflächenaktive Substanz vorhanden ist, so daß sich bei den nächsten Ausatmungsvorgängen bzw. beim Husten der Schleim aus den Bronchien löst.

## Patentansprüche

1. Therapiegerät (1) zur Unterstützung der Atmung und der Expektoration eines Patienten, mittels dem beim Aus- oder Einatmen ein oszillierender Luftwiderstand erzeugbar ist,
**dadurch gekennzeichnet,**
daß an einem Mundstück (11) ein elastisch verformbares Schlauchstück (21) vorzugsweise auswechselbar befestigt ist, das an seinem freien Ende (23) offen ausgebildet ist, und daß das Mundstück (11) in ein gekrümmt oder abgeknickt gestaltetes Rohrstück (31) eingesetzt ist, derart, daß das Schlauchstück (21) an dessen Innenmantelflache (34) anliegt.

2. Therapiegerät nach Anspruch 1,
**dadurch gekennzeichnet,**
daß das Mundstück (11) in Achsrichtung des Rohrstückes (31) verstellbar in diesem angeordnet ist.

3. Therapiegerät nach Anspruch 2,
**dadurch gekennzeichnet,**
daß zur axialen Verstellung und/oder zum Verdrehen des Mundstückes (11) auf dessen Außenmantelfläche mit Abstand zueinander angeordnete Ausnehmungen (13) in Form von Rillen (14), Kerben oder dgl. eingearbeitet sind, und daß an dem das Mundstück (11) aufnehmenden Ende (32) des Rohrstückes (31) ein oder mehrere gleichmäßig über den Umfang verteilt angeordnete, z. B. als Rastnasen (36) ausgebildete Vorsprünge (35) vorgesehen sind, die in die Ausnehmungen (13) eingreifen.

4. Therapiegerät nach einem oder mehreren der Ansprüche 1 bis 3,
**dadurch gekennzeichnet,**
daß das an dem Mundstück (11) angebrachte Schlauchstück (21) eine rechteckige Querschnittsfläche aufweist und in sich elastisch verformbar ist.

5. Therapiegerät nach einem oder mehreren der Ansprüche 1 bis 4,
**dadurch gekennzeichnet,**
daß das an dem Mundstück (11) angebrachte Schlauchstück (21) in dem an der Innenmantelfläche (34) des Rohrstückes (31) anliegenden Bereich eine Abwinkelung (24) aufweist.

6. Therapiegerät nach Anspruch 5,
**dadurch gekennzeichnet,**
daß das an dem Mundstück (11) angebrachte Schlauchstück (21) durch das axiales Zustellen und/oder durch Verdrehen des Mundstückes (11) in seinem Schwingungsverhalten veränderbar ist.

7. Therapiegerät nach einem oder mehreren der Ansprüche 1 bis 6,
**dadurch gekennzeichnet,**
daß in das freie Ende (33) des Rohrstückes (31) ein weiteres Mundstück (15) eingesetzt ist oder daß das freie Ende (33) des Rohrstückes (31) mit einer luftdurchlässigen oder teilweise offenen Verschlußkappe (37) versehen ist.

8. Therapiegerät nach Anspruch 7,
**dadurch gekennzeichnet,**
daß die Verschlußkappe (37) als Schalldämpfer ausgebildet ist.

## Claims

1. An apparatus for therapy (1) for supporting the breathing and expectoration of a patient, by means of which an oscillatory air resistance can be created during inhalation or exhalation,
**characterised in that,**
a flexible, deformable hose section (21) is fitted to a mouthpiece (11), with the hose section (21) being, in a preferred embodiment, exchangeable and an opening is produced at its free end (23), and that the mouthpiece (11) is inserted into a curved or bent tubular section (31) in such a way that the hose section (21) contacts the inside wall (34) of the tubular section (31).

2. The apparatus for therapy in accordance with claim 1,
**characterised in that,**
the mouthpiece (11) is fitted in the tubular section (31) so it is axially adjustable in relation to the tubular section (31).

3. The apparatus for therapy in accordance with claim 2,
**characterised in that,**
slots (13) in the form of grooves (14), notches or similar are produced at intervals in the outer wall of the mouthpiece (11) for axial movement and/or twisting of the mouthpiece (11), and that one or more projections (35), for example in the form of detent lugs (36), are arranged evenly around the circumference of the end (32) of the tubular section (31) which accommodates the mouthpiece (11), with the projections (35) engaging in the slots (13).

4. The apparatus for therapy in accordance with one or more of claims 1 to 3,
**characterised in that,**
the hose section (21) connected to the mouthpiece (11) has a rectangular cross-section and is capable of inherent flexible distortion.

5. The apparatus for therapy in accordance with one or more of claims 1 to 4,
**characterised in that,**
the hose section (21) connected to the mouthpiece (11) has a bend (24) in the area which contacts the inside wall (34) of the tubular section (31).

6. The apparatus for therapy in accordance with claim 5,
**characterised in that,**
the vibratory characteristics of the hose section (21) attached to the mouthpiece (11) can be altered by pushing it in axially and/or by turning the mouthpiece (11).

7. The apparatus for therapy in accordance with one or more of claims 1 to 6,
**characterised in that,**
a further mouthpiece (15) is inserted into the free end (33) of the tubular section (31) or that the free end (33) of the tubular section (31) is provided with an air-permeable or partially open sealing cap (37).

8. The apparatus for therapy in accordance with claim 7,
**characterised in that,**
the sealing cap (37) is designed as a muffler.

## Revendications

1. Appareil thérapeutique (1) servant à assister la respiration et l'expectoration d'un patient en créant une résistance d'air oscillante à l'aspiration et à l'expiration,
caractérisé en ce que
sur un bec (11), il est prévu, de préférence échangeable, un tuyau flexible élastique (21), dont l'extrémité libre (23) est ouverte et que le bec (11) est inséré dans un tuyau (31) courbé ou angulaire, de sorte que le tuyau flexible élastique (21) porte sur sa surface intérieure (34).

2. Appareil thérapeutique d'après la revendication 1,
caractérisé en ce que
le bec (11) est disposé dans le tuyau (31) de sorte qu'il se laisse ajuster en direction axiale de celui-ci.

3. Appareil thérapeutique d'après la revendication 2,
caractérisé en ce que
pour l'ajustement axial et/ou pour la rotation du bec (11), il est prévu sur sa surface extérieure et avec une certaine distance entre eux, des évidements (13) sous la forme de rainures (14), d'entailles ou de creux semblables et que sur l'extrémité (32) du tuyau (31) recevant le bec (11), il est prévu une ou plusieurs saillies (35) réparties uniformément sur le pourtour, conçues sous la forme de nez de crantage (36), qui s'engrènent dans les évidements (13).

4. Appareil thérapeutique d'après une ou plusieurs des revendications 1 à 3,
caractérisé en ce que
le tuyau flexible (21) prévu sur le bec (11) ait une section rectangulaire et qu'il se laisse déformer de manière élastique en soi.

5. Appareil thérapeutique d'après une ou plusieurs des revendications 1 à 4,
caractérisé en ce que,
à l'endroit portant sur la surface intérieure (34) du tuyau (31), le tuyau flexible (21) prévu sur le bec (11) forme un angle (24).

6. Appareil thérapeutique d'après la revendication 5,
caractérisé en ce que
l'oscillation du tuyau flexible (21) prévu sur le bec (11) se laisse varier par l'avance axiale et/ou en tournant le bec (11).

7. Appareil thérapeutique d'après une ou plusieurs des revendications 1 à 6,
caractérisé en ce que
dans l'extrémité libre (33) du tuyau (31), il est inséré un autre bec (15) ou que l'extrémité libre (33) du tuyau (31) est munie d'un capot de fermeture (37) perméable ou partiellement ouvert.

8. Appareil thérapeutique d'après la revendication 7,
caractérisé en ce que
le capot de fermeture (37) est conçu sous la forme d'un silencieux.
